# EUROPÄISCHE PATENTSCHRIFT

(11) **EP 1 423 089 B1**
(45) Veröffentlichungstag und Bekanntmachung des Hinweises auf die Patenterteilung: **20.05.2009**
(21) Anmeldenummer: 02743083.4
(22) Anmeldetag: 23.05.2002
(51) Int. Cl.: A61K 36/48, A61K 8/362, A61K 8/44, A61K 8/97, A61K 31/19, A61Q 19/00

(54) **KOSMETISCHE UND/ODER PHARMAZEUTISCHE ZUBEREITUNGEN ENTHALTEND EINEN EXTRAKT AUS PTEROCARPUS MARSUPIUM**
COSMETIC AND/OR PHARMACEUTICAL PREPARATIONS CONTAINING AN EXTRACT FROM PTEROCARPUS MARSUPIUM
PREPARATIONS COSMETIQUES ET/OU PHARMACEUTIQUES CONTENANT UN EXTRAIT DE PTEROCARPUS MARSUPIUM

(30) Priorität: 01.06.2001 EP 01401427
(43) Veröffentlichungstag der Anmeldung: 02.06.2004
(73) Patentinhaber: Cognis France, S.A.S., 31360 Boussens (FR)
(72) Erfinder: PAULY, Gilles, F-54000 Nancy (FR); JEANMAIRE, Christine, F-54000 Nancy (FR); PAULY-FLORENTINY, Muriel, F-54000 Nancy (FR)
(74) Vertreter: Gittinger, Andreas
(86) Internationale Anmeldenummer: PCT/EP2002/005659
(87) Internationale Veröffentlichungsnummer: WO 2002/098384

(56) Entgegenhaltungen:
- EP-A- 0 956 867

## Beschreibung

### Gebiet der Erfindung

Die Erfindung befindet sich auf dem Gebiet der Kosmetik und/oder Pharmazie und betrifft neue Mittel, die neben Pflanzenextrakten der Pflanze *Pterocarpus marsupium* Dicarbonsäuren und/oder deren Salze und/oder Aminosäuren enthalten sowie die Verwendung der Mittel als Pflegemittel für Haut und/oder Haare.

### Stand der Technik

Kosmetische Zubereitungen stehen dem Verbraucher heute in einer Vielzahl von Kombinationen zur Verfügung. Dabei wird nicht nur erwartet, dass diese Kosmetika einen bestimmten pflegenden Effekt zeigen oder einen bestimmten Mangel beheben, sondern immer häufiger wird nach Produkten verlangt, die mehrere Eigenschaften gleichzeitig aufweisen und somit ein verbessertes Leistungsspektrum zeigen. Ebenso darf der Anwender erwarten, dass die Zusammensetzung des Produktes eine optimale dermatologische Verträglichkeit besitzt, so dass auch empfindliche Verbraucher nicht mit Irritation reagieren. Darüber hinaus sollten die Mittel jedoch auch weitere Funktionen erfüllen, die zunehmend im Bereich der Pflege und insbesondere der Protektion liegen. Von besonderem Interesse sind Stoffe, die sowohl Wirkstoffe darstellen, die für Haut und Haare beispielsweise pflegende, vor Alterserscheinungen schützende, revitalisierende Eigenschaften vermitteln als auch gleichzeitig die technischen Eigenschaften des kosmetischen Produktes, wie Lagerstabilität, Lichtstabilität und Formulierbarkeit positiv beeinflussen oder zumindest nicht verschlechtern. Hierbei sind zusätzlich eine gute Hautverträglichkeit und besonders der Einsatz natürlicher Produkte beim Kunden gefragt. Daneben ist es wünschenswert, durch Kombination bereits bekannter Wirkstoffe, oder durch auffinden neuer Einsatzgebiete bereits bekannter Substanzklassen deutlich bessere Produkte zu erhalten. Die Kombination bereits bekannter Wirkstoffe führt nicht selten dazu, dass es zu positiv synergistischen Effekten kommt und die Konzentration der einzusetzenden Wirkstoffe vermindert werden kann. Ein Nachteil besteht hier allerdings häufig darin, das eine Kombination von Wirkstoffen erst dann erhalten wird, wenn unterschiedliche Pflanzenextrakte gleichzeitig in unterschiedlichen Mengenverhältnissen verwendet werden.

Extrakte von Pflanzen und deren Inhaltstoffe finden immer häufiger Einsatz in der Kosmetik und Pharmakologie. Pflanzenextrakte werden seit vielen Jahren in den unterschiedlichsten Kulturen für medizinische aber auch bereits für kosmetische Zwecke genutzt. Oftmals waren für diese Pflanzenextrakte nur ganz bestimmte einzelne Wirkungen bekannt und das Einsatzgebiet sehr eingeschränkt.

Besonders in den Grenzgebieten zwischen Kosmetik und Pharmazie wächst ein großes Interesse an Pflegestoffen, die pharmazeutische Wirksamkeiten zeigen mit sehr geringen Nebenwirkungen. Werden diese Pflegestoffe in kosmetischen Mitteln angeboten so bietet es dem Verbraucher die Möglichkeit bequem und ohne großen Aufwand Mangelerscheinungen zu beheben oder zu verhindern.

### Beschreibung der Erfindung

Die Aufgabe der vorliegenden Patentanmeldung hat darin bestanden, Extrakte aus nachwachsender Rohstoffen für die kosmetische und/oder pharmazeutische Anwendung zur Verfügung zu stellen, die gleichzeitig eine vielseitige Anwendung als Pflegemittel in den unterschiedlichsten Bereichen der Kosmetik und/oder Pharmazie ermöglichen.

Eine weitere Aufgabe der vorliegenden Patentanmeldung hat darin bestanden kosmetische und/oder pharmazeutische Mittel bereitzustellen, die neben pflegenden und schützenden Eigenschaften vor allem vorbeugende und heilende Wirkung zeigen, insbesondere bei Haarausfall und bei Alterserscheinungen der Haut sowie reaktivierend und revitalisierend wirken können.

Gegenstand der Erfindung sind kosmetische und/oder pharmazeutische Mittel, enthaltend
(a) eine wirksame Menge eines Extraktes aus *Pterocarpus marsupium* und
(b) Dicarbonsäuren und/oder deren Salze und/oder Aminosäuren.

Überraschenderweise wurde gefunden, dass durch den Einsatz von Extrakten aus Pterocarpus marsupium in Kombination mit Dicarbonsäuren und/oder deren Salze und/oder in Kombination mit Aminosäuren Produkte erhalten werden, die gleichzeitig gute pflegende und schützende Eigenschaften für die Haut, insbesondere der Kopfhaut zeigen sowie eine hohe Hautverträglichkeit besitzen. Die so erhaltenen Mittel zeichnen sich durch besonders gute Effekte in der Hautkosmetik aus. Sie zeigen vorbeugende und heilende Wirkung bei Haarausfall und bei Alterserscheinungen der Haut und eine revitalisierenden und reaktivierende Aktivität auf die Haut.

Diese mehrfachen Einsatzgebiete der erfindungsgemäßen Mittel enthaltend. Extrakte aus dem nachwachsendem Rohstoff der Pflanze *Pterocarpus marsupium* in Kombination mit Dicarbonsäuren und/oder deren Salze und/oder Aminosäuren macht es für den Markt und für den Verbraucher sehr attraktiv. Die komplexe Aufgabe der Erfindung konnte somit durch den Einsatz dieser Mittel gelöst werden.

Unter dem Begriff Pflanze im Sinne der vorliegenden Anmeldung sind sowohl ganze Pflanzen als auch Pflanzenteile (Blätter, Wurzeln, Blüten) sowie deren Gemische zu verstehen.

### Pterocarpus marsupium

Die erfindungsgemäßen Extrakte werden aus Pflanzen der Familie **Pterocarpus** gewonnen. Konkrete Beispiele sind die Extrakte von *Pterocarpus macrocarpus, P. santalinus* (Rotes Sandelholz), *P*. *angolensis, P. indicus, P. soyaauxii;* aus anwendungstechnischer Sicht hat sich jedoch vor allem der Extrakt von *Pterocarpus marsupium* bewährt. Bei der Pflanze *Pterocarpus marsupium* handelt es sich um Pflanzen die reichlich in belaubten Wäldern Süd-, West-, und Ost-Indiens und auf Ceylon wachsen. Sie erreichen eine-Höhe von 15 bis 25 Metern. Die dunkelbraune bis graue Rinde zeigt große Risse und sondert nach Verletzung eine rötliche, gummiartige Substanz (genannt "kino-gum") ab. Die Blätter werden 15 bis 25 cm groß und die Blüten erscheinen gelb. Die Früchte sind flach und rund und enthalten ein bis zwei kleine Samen. Der Kern des Holzes ist hart und gold bis rot-braun.

In der traditionellen indischen Medizin wird das Holz und die Rinde u.a. als Anti-diabetikum und Antidiarrethikum eingesetzt. Sie zeigen weiterhin astringierende und anti-inflammatorische Wirkung. Die Blätter werden u.a. als Futtermittel verwendet, aber auch zur Wundheilung und speziell zur Heilung von Hautkrankheiten. Die gummi-artige Substanz aus der Rinde wird u.a. verwendet gegen Blutkrankheiten. Über den Einsatz von Sandelholzextrakt im allgemeinen berichtet J.Verghese in Cosm.Toil.101(4), 69 (1986). Hauptbestandteile der Pterocarpus-Extrakte sind Santaline, Sterole und Flavonderivate. In diesem Zusammenhang sei weiterhin beispielsweise auf das französische Patent FR-B1 2483228 (Pierre Fabre) verwiesen, aus dem Haarfärbemittel auf Basis von Pterocarpus-Extrakten bekannt sind. Der Einsatz von Pterocarpus-Extrakten als oxidative Haarfarbstoffe wird auch in der japanischen Patentanmeldung JP-A1 Hei 10/182372 (Lion) beschrieben. Gegenstand der internationalen Patentanmeldung WO 98/44902 (L'Oréal) sind schließlich Selbstbräunungsmittel mit einem Gehalt an Sandelholzextrakt und einem kosmetisch akzeptablen Ölkörper.

### Extraktion

Die Herstellung der erfindungsgemäß einzusetzenden Extrakte erfolgt durch übliche Methoden der Extraktion von Pflanzen bzw. Pflanzenteilen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf Hagers Handbuch der Pharmazeutischen Praxis, (5. Auflage, Bd. 2, S. 1026-1030, Springer Verlag, Berlin-Heidelberg-New-York 1991) verwiesen. Als Ausgangsmaterial können frische oder getrocknete Pflanzen oder Pflanzenteile eingesetzt werden, üblicherweise wird jedoch von pflanzen und/oder Pflanzenteilen ausgegangen, die vor der Extraktion mechanisch zerkleinert und gegebenenfalls entfettet werden. Hierbei eignen sich alle dem Fachmann bekannten Zerkleinerungsmethoden, als Beispiel sei die Zerkleinerung mit einem Klingen enthaltenen Gerät genannt.

Als Lösungsmittel für die Durchführung der Extraktionen können vorzugweise Wasser, organische Lösungsmittel oder Gemische aus organischen Lösungsmitteln und Wasser, insbesondere niedermolekulare Alkohole, Ester, Ether, Ketone oder halogenhaltige Kohlenwasserstoffe mit mehr oder weniger hohen Wassergehalten (destilliert oder nicht destilliert) vorzugsweise wässrig, alkoholische Lösungen mit mehr oder weniger hohen Wassergehalten, verwendet werden. Besonders bevorzugt ist die Extraktion mit destilliertem Wasser, Methanol, Ethanol, Propanol, Butanol und deren Isomere, Aceton, Propylenglycolen, Polyethylenglycolen, Ethylacetat, Dichlormethan, Trichlormethan sowie, Mischungen hieraus. Die Extraktion erfolgt in der Regel bei 20 bis 100°C. bevorzugt bei 80 bis 100 °C, insbesondere bei 80 bis 90 °C. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, beispielsweise durch Filtration, unterzogen werden. Die Extraktion kann bis zu jedem gewünschten Extraktionsgrad erfolgen, wird aber gewöhnlich bis zur - Erschöpfung durchgeführt. Die vorliegenden Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden. Typische Ausbeuten (= Trockensubstanzmenge des Extraktes bezogen auf eingesetzte Rohstoffmenge) bei der Extraktion getrockneter Pflanzen oder getrockneter Pflanzenteile gegebenenfalls entfettet, liegen im Bereich von 1 bis 20, vorzugsweise 2 bis 15, insbesondere 3 bis 10 Gew.-%.

Die Einsatzmenge der Pflanzenextrakte in den genannten Mitteln richtet sich nach der Konzentration der einzelnen Inhaltstoffe und nach der Art der Anwendungen der Extrakte. Die Gesamtmenge des Pflanzenextraktes, der in den erfindungsgemäßen Zubereitungen enthalten ist, beträgt in der Regel 0,001 bis 25 Gew.-%, vorzugsweise 0,005 bis-10 Gew.-%, insbesondere 0,01 bis 5 Gew.-% bezogen auf die Endzubereitung, mit der Maßgabe, dass sich die Mengenangaben mit Wasser und gegebenenfalls weiteren Hilfs- und Zusatzstoffen zu 100 Gew.-% addieren.

Die erfindungsgemäßen Extrakte enthalten einen Wirkstoffgehalt in den Extrakten von 5 bis 100 Gew.-%, vorzugsweise 10 bis 95 Gew.%, insbesondere 20 bis 80 Gew.-%. Der Wirkstoffgehalt im Sinne der Erfindung bezeichnet die Summe aller im Extrakt vorhandenen Wirkstoffe bezogen auf das Trockengewicht des Extraktes.

Wirkstoff im Sinne der Erfindung bezieht sich auf die im Extrakt enthaltenen Inhaltstoffe auch wenn deren Gehalt und Identität mit herkömmlichen; dem Fachmann bekannten Methoden noch nicht nachzuweisen sind. Unter Wirkstoffe im Sinne der Erfindung sind weiterhin alle im Extrakt erhaltenen Inhaltsstoffe zu verstehen, deren Wirkung entweder bereits bekannt ist, oder deren Wirkung mit herkömmlichen, dem Fachmann bekannten Methoden noch nicht nachgewiesen werden konnte.

Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers.

Der Gesamtanteil an Hilfs- und Zusatzstoffe kann 1 bis 50, vorzugsweise 5 bis 40 Gew.-% - bezogen auf die Endzubereitung der kosmetischen und/oder dermatologischen Zubereitungen - betragen. Die Herstellung der Zubereitungen kann durch übliche Kalt - oder Heißprozesse erfolgen; vorzugsweise arbeitet man nach der Phaseninversionstemperatur-Methode.

### Extrakte:

In einer bevorzugten Ausführungsform der Erfindung enthalten die Extrakte der Pflanze *Pterocarpus marsupium* in den erfindungsgemäßen Mitteln ganz oder überwiegend als Wirkstoffe Flavonderivate, insbesondere Kämpferol und Kämpferol-Derivate, 7-Hydroxyflavanone, Liquiritigenine, Isoliquiritigenine, 7,44-Dihydroxyflavone, Marsupine, Pterosurpine und (-)-Epicatechin. Aber auch Tannine sowie freie Phenolsäure, insbesondere p-Hydroxyphenylmilchsäure können in den Extrakten als Wirkstoffe enthalten sein. Die Extrakte sind je nach gewähltem Ausgangsmaterial und nach gewählter Extraktionsmethode unterschiedlich zusammengesetzt.

Im Sinne der vorliegenden Erfindung sind unter **Flavonderivaten** solche zu verstehen, die sich aus der Pflanze Pterocarpus marsupium isolieren lassen. Im Besonderen handelt es sich um Stoffe, die Hydrierungs-, Oxidations- oder Substitutionsprodukte des 2-Phenyl-4H-1-benzopyrans darstellen, wobei eine Hydrierung in der 2,3-Stellung des Kohlenstoffgerüsts bereits vorliegen kann, eine Oxidation in der 4-Stellung bereits vorliegen kann, und unter Substitutionsprodukte der Ersatz eines oder mehrerer Wasserstoffatome durch Hydroxy- oder Methoxy-Gruppen zu verstehen ist. Bei dieser Definition sind also Flavane, Flavan-3-ole (Catechine), Flavan-3,4-diole (Leukoanthocyanidine), Flavone, Flavonole und Flavanone im herkömmlichen Sinn eingeschlossen. Als besonders bevorzugte Flavonderivate isoliert aus der Pflanze Pterocarpus marsupium gelten Kämpferol und Kämpferol-Derivate, 7-Hydroxyflavanone, Liquiritigenine, Isoliquiritigenine, 7,44-Dihydroxyflavone, Marsupine, Pterosurpine und (-)-Epicatechin.

Die erfindungsgemäßen Mittel enthalten neben den Extrakten aus *Pterocarpus marsupium* Dicarbonsäuren und/oder deren Salze und/oder Aminosäuren. Besonders bevorzugt ist eine Kombination aus Extrakt aus Pterocarpus marsupium, Salzen der Dicarbonsäure und Aminosäuren.

### Dicarbonsäuren:

In einer bevorzugten Ausführungsform enthalten die erfindungsgemäßen Mittel Dicarbonsäuren die ausgewählt sind aus der Gruppe, die gebildet wird von Oxalsäure, Malonsäure, Bernsteinsäure und Glutarsäure. In einer weiteren besonderen Ausführungsform enthalten die erfindungsgemäßen Mittel Salze dieser Dicarbonsäuren, die ausgewählt sind aus der Gruppe die gebildet wird aus Alkali- und Erdalkalisalzen, insbesondere aus Natrium-, Kalium, Magnesium- und Calciumsalzen. Besonders bevorzugt ist Natriumsuccinat, das Natriumsalz der Bernsteinsäure.

### Aminosäuren:

In einer bevorzugten Ausführungsform enthalten die erfindungsgemäßen Mittel Aminiosäuren, die ausgewählt sind aus der Gruppe, die gebildet wird von Glycin, Alanin, Leucin, Isoleucin, Serin, Threonin, Cystein, Asparaginsäure, Glutaminsäure, Asparagin, Glutamin, Phenylalanin, Tyrosin, Methionin, Valin, Prolin, Lysin und Histidin. Besonders bevorzugt sind Aminosäuren, die ausgewählt sind aus der Gruppe, die gebildet wird von Histidin, Asparaginsäure und Glutaminsäure, insbesondere bevorzugt ist Glutaminsäure.

Die erfindugnsgemäßen Mittel enthalten vorzugsweise und in einer besonderen Ausführungsform
(a) 0,001 bis 25 Gew.-% Extrakt und
(b) 0,001 bis 15 Gew.-% Dicarbonsäuren und/oder deren Salze
mit der Maßgabe, dass sich gegebenenfalls die Mengenangaben mit Wasser und/oder weiteren Hilfs- und Zusatzstoffen zu 100 Gew.-% ergänzen.

In einer weiteren bevorzugten Ausführungsform enthalten die Mittel vorzugsweise
(a) 0,001 bis 25 Gew.-% Extrakt und
(b) 0,0005 bis 10 Gew.-% Aminosäuren
mit der Maßgabe, dass sich gegebenenfalls die Mengenangaben mit Wasser und/oder weiteren Hilfs- und Zusatzstoffen zu 100 Gew.-% ergänzen.

Insbesondere enthalten die erfindugnsgemäßen Mittel in einer besonderen Ausführungsform
(a) 0,001 bis 25 Gew.-% Extrakt und
(b) 0,001 bis 15 Gew.-% Dicarbonsäuren oder deren Salze und 0,0005 bis 10 Gew.-% Aminosäuren
mit der Maßgabe, dass sich gegebenenfalls die Mengenangaben mit Wasser und/oder weiteren Hilfs- und Zusatzstoffen zu 100 Gew.-% ergänzen.

Die Kombination von *Pterocarpus marsupium-Extrakten* mit Dicarbonsäuren und/oder deren Salze und/oder Aminosäuren, sowie insbesondere die aktiven Wirkstoffe aus den Extrakten, die Flavonderivate weisen eine Vielzahl von kosmetischen und pharmazeutischen Wirkungen auf.

Weitere Gegenstände der Erfindung betreffen daher Mittel enthaltend
(a) eine wirksame Menge eines Extraktes aus *Pterocarpus marsupium* und
(b) Dicarbonsäuren und/oder deren Salze und/oder Aminosäuren

➢ gegen Haarausfall, insbesondere gegen androgene Alopezie;
➢ als Mittel gegen die Hautalterung;
➢ als UV/IR-Lichtschutzmittel
➢ als Mittel gegen die Schädigung von Fibroblasten und/oder Keratinocyten durch UV-Strahlung, insbesondere durch UV-B-Strahlung;
➢ als Mittel gegen oxidativen Stress für Haut und/oder Haare

Obwohl die synergistisch wirkende Kombination der *Pterocarpus marsupium-*Extrakten mit Dicarbonsäuren und/oder deren Salze und/oder Aminosäuren bevorzugt ist und besonders gute Effekte in den genannten Verwendungen zeigen, weisen die Extrakte aus *Pterocarpus marsupium* auch ohne die synergistsiche Kombination eine Vielzahl von kosmetischen und pharmazeutischen Wirkungen auf. Weitere Gegenstände der Erfindung betreffen daher *Pterocarpus marsupium-*Extrakte
➢ gegen Haarausfall, insbesondere gegen androgene Alopezie
➢ als Mittel gegen die Hautalterung;
➢ als UV/IR-Lichtschutzmittel
➢ als Mittel gegen die Schädigung von Fibroblasten und/oder Keratinocyten durch UV-Strahlung, insbesondere durch UV-B-Strahlung;.
➢ als Mittel gegen oxidativen Stress für Haut und/oder Haare

### Pflegemittel:

Als Pflegemittel im Sinne der Erfindung sind Pflegemittel für die Haare und die Haut zu verstehen, insbesondere die Kopfhaut. Diese Pflegemittel schließen unter anderem stimulierende, heilende und aufbauende Wirkung mit ein. Als Pflegemittel im Sinne der Erfindung sind bevorzugt solche Pflegemittel zu verstehen, die gegen Haarausfall wirken, die einen stimulierenden Effekt auf die Hautzellen und deren Funktionen besitzen sowie weiterhin aufbauende Wirkung für Haut und Haare zeigen. Sie wirken weiterhin vorbeugend gegen Umwelteinflüsse für die Haut. Weiterhin sind im Sinne der Erfindung bevorzugt Pflegemittel als solche zu verstehen, die verschiedene Erkrankungen der Haut mit ihren unterschiedlichen Auswirkungen auf Aussehen und Funktion der Haut entweder verbessern oder heilen können. Prinzipiell kann man die erfindungsgemäßen Extrakte in allen kosmetischen Produkten zur topischen Verwendung einsetzen. Beispiele für kosmetische Produkte sind in ihren Formulierungen in den Tabelle 5 bis 8 beschrieben.

Die vorliegenden Erfindung schließt die Erkenntnis ein, dass durch das Zusammenwirken der Inhaltsstoffe der Pflanzenextrakte, insbesondere der oben genannten, besonders wirkungsvolle kosmetische Mittel erhalten werden.

Die erfindungsgemäßen Zubereitungen zeigen eine hervorragende hautpflegende Wirkung bei gleichzeitig hoher Hautverträglichkeit. Außerdem zeigen sie eine gute Stabilität, insbesondere gegenüber oxidativer Zersetzung der Produkte.

Die Begriffe Zubereitungen, Endzubereitungen und Mittel sind im Sinne der Erfindung mit dem Begriff Pflegemittel gleichzusetzen.

Aktivsubstanz im Sinne der Erfindung bezieht sich auf den Anteil an Substanzen sowie Hilfs- und Zusatzstoffen, die in den Zubereitungen enthaltend sind, mit Ausnahme des zusätzlich hinzugefügten Wassers.

### Haarausfall

Die erfindugsgemäßen Mittel und Extrakte aus *Pterocarpus marsupium* wirken gegen Haarausfall und insbesondere gegen androgenetisch bedingtem Haarausfall, auch als androgene Alopezie bezeichnet.

Haarausfall wird physiologisch-normal mit 30-100 Haaren/Tag angegeben.

Der Haarausfall, der zur Bildung einer Glatze bei Männern und bei Frauen führt, ist überwiegend hormonellen Ursprungs (sog. androgene Alopezie), wobei allerdings auch die Erbanlage eine Rolle spielt. Glatzenbildung beginnt beim Mann mit dem Erscheinen von "Geheimratsecken". Die androgene Alopezie ist charakterisiert durch einige Änderungen in den Haarfollikeln. Diese Änderungen schliessen eine fortschreitende Verkleinerung der Haarfollikeln, eine Verkürzung der Wachstumsphase der Haare (Anagen-Phase) und eine Verlängerung der Ruhe-Phase (Telogen-Phase) mit ein. Daraus lässt sich ableiten, dass es sich bei der androgenen Alopezie um ein vielschichtiges Phänomen handelt. Die unterschiedlichsten Untersuchungen wurden bereits durchgeführt um diese phänomene zu reduzieren oder zu verhindern.

So untersuchte beispielsweise Reygagne et al. den Einfluss von 5-alpha-Reductase-Inhibitoren **(**Annales Dermatol. Venerol. 3, 1998**)** auf die Alopezie und Lachgar et al. versuchten durch Minoxidil die Expression von VEGF (vascular endothelial growth factor) zu erhöhen und so eine vermehrte Bildung von Blutgefäßen in den Haarfollikeln zu erreichen **(**British Journal of dermatology, 138, 1998, 407-411**).**In der FR 2724561 wird beschrieben, dass durch die Verwendung von Inhibitoren der Lysyl-Hydroxylase (ein Enzym der Kollagen-Ausbildung) Alopezie bekämpft werden soll.

Von der androgenen Alopezie sind die krankhaften Formen des Haarausfalls (Effluvium) zu unterscheiden, wie sie u. a. bei der Alopecia areata, einer Krankheit mit vermutlich durch Autoimmunität hervorgerufenen kahlen Hautstellen oder bei verschiedenen Infektionskrankheiten (Syphilis, Pilzerkrankungen) auftreten.

Telogener Haarausfall kann Folge von Infektionen sein oder nach Gravidität auftreten. Auch krankhaft modifiziertes Wachstum der Haare kann Ursache sein. Ferner ist Haarausfall bei Mangelzuständen, nach Medikamentenapplikation (z.B. von Cytostatika, Antikoagulantien) und bei Vergiftungen (z.B. mit Thallium-Salzen) möglich.

Es konnte gezeigt werden, dass die enzymatische Glykosylierung von Proteinen in der Haut sowie der Kopfhaut nachweisbar ist, jedoch ist die Rate an AGE (advanced glycating endproducts) gemessen bei ein und derselben Person höher in den Geweben unbehaarter als denen behaarter Flächen.

Die erfindungsgemäßen Mittel und Extrakte aus *Pterocarpus marsupium* besitzen eine Anti-Glykosylierungs-Aktivität, insbesondere wirken sie gegen die Glykosylierung von kutanen Proteinen und bevorzugt gegen die Glykosylierung von Kollagen. Im Jahre 1981 **(**Science, 1981, 211, 491-493**)** beschrieb A. Cerami die Glykosylierung von Proteinen oder nichtenzymatische Glykosylierung im Unterschied zur enzymatischen Glykosylierung durch Glukosyltransferase und erwähnte die mögliche Rolle dieser Glykosylierung bei der Alterung von Geweben. Der biochemische Mechanismus dieser Reaktion ist gut bekannt **(**Borel J.P. et al., CR biologie prospective, 145-149,1993**)** und läuft in zwei Phasen ab:

In einer frühen Phase reagieren zunächst reduzierende Zucker (Glucose, Fructose) mit den end- oder seitenständigen Aminofunktionen der im Gewebe enthaltenen Proteine zu sogenannten Schiffschen Basen. Diese Verbindungen werden dann durch Amadori-Umlagerung zum Ketoamin stabilisiert.

Die Ketoaminfunktionen werden dann in einer späten Phase in Gegenwart von Sauerstoff zur Desoxyonose oxidiert und reagieren mit zu anderen Proteinen (Albumin, Lipoproteinen, Immunoglobulin) gehörigen anderen basischen Aminosäuren wie Arginin oder Lysin. Dabei bilden sich Komplexe, die letztendlich über Pentosidin- oder 2-Furoyl-1,4-imidazol-Cyclen verbrückt sind. Durch diese Verbrückungen ergeben sich als komplexe und sehr stabile Endprodukte die sogenannten AGE ("Advanced Glycosylation Endproducts") oder fortgeschrittene Produkte der Glykosylierung. Diese späte Phase ist sehr langsam und irreversibel.

Die Glycosylierung der Proteine führt zur Bildung von inter- und intramolekularen Brücken bei langsam erneuerten Proteinen und schließlich zur Braunfärbung und Unlöslichkeit dieser Proteine. Die Glykosylierting betrifft insbesondere die Proteine in den extrazellulären Matrizes, deren Erneuerung langsam ist.

Im Bereich der Haut handelt es sich bei den durch die Glycosylierung geschädigten Proteinen insbesondere um Fibronektin, Laminin, Elastin, verschiedene Kollagentypen, Elastin, GAGs und kleine Proteoglucane.

Die AGE führen zu verschiedenen Beschwerden:
15. da sie voluminös sind, bleiben die Moleküle, die sie tragen, nur schwerlich an ihrem normalen Platz,
16. die glycosylierten Moleküle büßen ihre Flexibilität ein (Gewebeversteifung),
17. die glycosylierten Moleküle können gegenüber Enzymen, die deren Erneuerung gewährleisten, widerstandsfähiger werden und so Flächen aus amorphen Substanzen bilden.

In normaler Haut werden die glykosylierten Proteine auf metabolischem und zellulärem Wege eliminiert, insbesondere durch Abbau durch Makrophagen, was eine Neugestaltung der Dermis induziert.

Diese Eliminierung läßt jedoch mit zunehmendem Alter nach, was eine Anreicherung dieser glykosylierten Proteine und eine beschleunigte und verstärkte Alterung der Dermis zur Folge hat, wofür die Summe mehrerer Phänomene verantwortlich ist:
18. Beständigkeit gegenüber Emeuerungsproteasen und Abnahme der Fibrillogenese und damit der Erneuerung von Kollagen, Verminderung seiner Filterwirkung in der extrazellulären Matrix und insbesondere am Dermis-Epidermis-Übergang nach Fixierung von Fremdproteinen (LDL, Cholesterin, Albumin), was zu Verdickung führt,
19. die glykosylierten Proteine stellen eine potentielle Quelle freier Sauerstoffradikale dar. Dieses durch UV-A verstärkte Phänomen führt zum Kollagenabbau,
20. Aktivierung von unspezifischen, schädlichen Proteasen,
21. Aktivierung von Makrophagen und Ausschüttung von Cytokinen (TNF-alpha),
22. schließlich Entzündung mit anschließender Fibrose und Lipofuszin-Ablagerung.

Die obigen Ausführungen sind für die Verwendung von Substanzen mit Anti-Glykosylierungs-Aktivität, insbesondere bei der Bekämpfung und Prävention der Haut- und insbesondere der Haaralterung in der Kosmetik, von Interesse. Die Unterdrückung der nichtenzymatischen Glykosylierung stellt unter anderem ein wichtiges Ziel zur Verhinderung von Haarausfall dar.

Studien haben gezeigt, dass eine erhöhte Exposition der Haut mit UV-Strahlung zu einer Erhöhung der Glykosylierung einiger Proteine führt. Da die Kopfheut einer extrem hohen Belastung mit UV-Strahlung ausgesetzt ist und die Glykosylierung direkt mit dem Fortschreiten des Haarausfalls korreliert, können Mittel, die einer Glykosylierung entgegenwirken direkt gegen Haarausfall eingesetzt werden. Zeigen diese Mittel wie es für die erfindungsgemäßen Mittel nachgewiesen wurde zusätzlich noch UV/IR-Lichtschutzwirkung, so erfüllen sie hier den gewünschten vielfältigen Effekt.

Ein weiterer Effekt, der zur Erhöhung der Glykosylierung führen kann, ist eine Verringerung von Glutathion. Die erfindungsgemäßen Mittel und Extrakte aus *Pterocarpus marsupium* zeigen eine stimulierend Wirkung auf die Synthese von Glutathion. Glutathion ist ein wichtiges Tripeptid für den Metabolismus, den Transport und den Schutz der Zelle. Es ist bekannt, dass der Gehalt an Glutathion in den Haarfollikeln mit dem Alter abnimmt. Weiterhin wurde bereits untersucht, dass der Gehalt an Glutathion in den Kopfhautzellen, die von Haarausfall betroffen waren niedriger war als in den noch behaarten Bereichen der Kopfhaut **(**Giralt et al. J. Invest. Dermatol. 107, 1996, 154-158**).** Die stimulierende Wirkung der erfindungsgemäßen Mittel auf die Synthese von Glutathion kann also indirekt zu einer Verminderung von Haarausfall führen.

Die erfindungsgemäßen Mittel wirken weiterhin anti-inflammatorisch. Die Verwendung der erfindungsgemäßen Mittel als anti-inflammatorische Additive ist prinzipiell für alle kosmetischen und/oder pharmazeutischen Pflegemittel möglich, die bei Entzündungen der Haut und damit in der Hautpflege eingesetzt werden. Als anti-inflammatorisches Pflegemittel sind im Sinne der Erfindung die Art der Pflegemittel zu verstehen, die eine Entzündung der Haut heilen können oder die einer Entzündung vorbeugen können. Die Entzündungen können dabei die unterschiedlichsten Ursachen aufweisen.

Die erfindungsgemäßen Mittel und Extrakte aus *Pterocarpus marsupium* reduzieren weiterhin die Bildung von Interieukin-1-alpha (1'IL-α).

1'IL-α ist ein zu den Cytokinen gehörender Mediator-Stoff des Immunsystems, der auf Wachstum (als Wachstumsfaktoren), Differenzierung und Aktivität von Zellen des Immunsystems Einfluß nehmen kann (Immunmodulaton).

Speziell ist 1'IL-α beim Wachstum der Haarfollikel in der Anagen-Phase beteiligt und initiert vermutlich den Übergang in die Katagen-Phase. Studien haben gezeigt, dass 1'IL-α das Haarwachstum inhibieren kann. Eine erhöhte Konzentration an 1'IL-α führt zu entzündlichen Reaktionen. Es gibt also einen Zusammenhang zwischen der Konzentration an 1'IL-α, dem Haarausfall und entzündlichen Reaktionen. Durch die erfindungsgemäßen Mittel und durch Extrakte aus *Pterocarpus marsupium* wird die Bildung von 1'IL-α reduziert und damit indirekt einer entzündlichen Reaktion vorgebeugt und unter anderem Haarausfall verhindert.

Die erfindungsgemäßen Mittel und Extrakte aus *Pterocarpus marsupium* wirken weiterhin gegen Hautalterung und können zur vorbeugenden oder heilenden Behandlung von Alterserscheinungen der Haut verwendet werden. Eine andere Bezeichnung für diese Art der Pflegemittel ist auch an-ageing Mittel. Zu diesen Alterserscheinungen zählen beispielsweise jede Art der Fältchen- und Faltenbildung. Die Behandlungen schließen eine Verlangsamung von Altersprozessen der Haut mit ein. Die Alterserscheinungen können die unterschiedlichsten Ursachen aufweisen. Der Alterungsprozeß kann durch UV-Strahlung induziert werden, man spricht dabei vom "Photo-Aging". In einer besonderen Ausführungsform der Erfindung werden diese Pflegemittel zur Behandlung von durch UV-Strahlung induzierten Alterserscheinungen der Haut eingesetzt.

Die erfindungsgemäßen Mittel und Extrakte aus *Pterocarpus marsupium* wirken als UV-Lichtschutzmittel.

### Sonnenschutzmittel bzw. UV-Lichtschutzmittel

Als Sonnenschutzmittel bzw. UV-Lichtschutzmittel im Sinne der Erfindung werden Lichtschutzmittel bezeichnet, die für den Schutz der menschlichen Haut gegenüber schädigenden Einflüssen der direkten und indirekten Strahlung der Sonne nützlich sind. Die für die Hautbräunung verantwortliche Ultraviolettstrahlung der Sonne unterteilt man in die Abschnitte UV-C (Wellenlängen 200-280 nm), UV-B (280-315 nm) u. UV-A (315-400 nm).

Die Pigmentierung normaler Haut unter dem Einfluss der Sonnenstrahlung, d. h. die Bildung von Melaninen, wird durch UV-B und UV-A unterschiedlich bewirkt. Bestrahlung mit UV-A-Strahlen ("langwelligem UV") hat die Dunkelung der in der Epidermis bereits vorhandenen Melanin-Körper zur Folge, ohne dass schädigende Einflüsse zu erkennen sind. Anders bei dem sog. "kurzwelligen UV" (UV-B). Dieses bewirkt die Entstehung von sog. Spätpigment durch Neubildung von Melanin-Kömem. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) u. gar Brandblasen führen kann.

Diese weiteren UV- Lichtschutzfaktoren sind beispielsweise bei Raumtemperatur flüssig oder kristallin vorliegenden organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind bevorzugt zu nennen:
➢ 3-Benzylidencampher bzw. 3-Benrylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester,
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester,
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester,
➢ Triazinderivate, wie z.B. 2,4,6-Trianilino-(p-carbo-2'-ethyl-1'-hexyloxy)1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen insbesondere in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UVA-Filter kommen insbesondere Derivate des Benzoylmethans in Frage, wie beispielsweise 1-(4'-tert.Butytphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beispielsweise beschrieben in der DE 19712033 A1 (BASF). Die UV-A und UV-B-Filter können selbstverständlich auch in Mischungen eingesetzt werden. Besonders günstige Kombinationen bestehen aus den Derivaten des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Dimethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**)** sowie Parfümerie und Kosmetik 3 (1999), Seite 11ff zu entnehmen.

Die erfindungsgemäßen Mittel und Extrakte aus *Pterocarpus marsupium* werden wieterhin gegen die Schädigung von Fibroblasten und/oder Keratinocyten durch UV-Strahlung verwendet.

UVB-Strahlen lösen durch Aktivierung eines Enzyms, nämlich Phospholipase A2 oder PLA2 eine Entzündung aus. Diese Entzündung (Erythem, Ödem) wird durch die Entfernung von Arachidonsäure aus den Phospholipiden der Plasmamembran durch die Phospholipase ausgelöst. Arachidonsäure ist die Vorstufe der Prostaglandine, die eine Entzündung und eine Zellmembranschädigung verursachen; die Prostaglandine E2 (= PGE2) werden durch die Cyclooxygenase gebildet. Der Grad der Freisetzung des Cytoplasmaenzyms LDH (Lactat Dehydrogenase) in humanen Keratinocyten dient als Marker für eine Zellschädigung.

Die erfindungsgemäßen Mittel reduzieren den Effekt von UVB-Strahlung auf die Anzahl an Keratinocyten und auf den Gehalt an freigesetzte LDH. Die Mittel und Extrakte zeigen demnach die Fähigkeit, die durch UVB-Strahlung hervorgerufene Schädigung an Zellmembranen zu reduzieren.

Die erfindungsgemäßen Mittel und Extrakte aus *Pterocarpus marsupium* können verwendet werden gegen oxidativen Stress für Haut und/oder Haar. Gegen oxidativen Stress können neben den erfindungsgemäßen Mitteln und Extrakten aus *Pterocarpus marsupium* weiterhin Antioxidantien und Radikalfänger eingesetzt werden.

Antioxidantien sind in der Lage, die unerwünschten, durch Sauerstoff-Einwirkungen und andere oxidative Prozesse bedingten Veränderungen in den zu schützenden Stoffen zu hemmen oder zu verhindern. Die Wirkung der Antioxidantien besteht meist darin, dass sie als Radikalfänger für die bei der Autoxidation auftretenden freien Radikale wirken.

Eine mögliche Anwendung der Antioxidantien zum Beispiel in kosmetischen und/oder pharmazeutischen Zubereitungen, ist die Anwendung als sekundäre Lichtschutzmittel, weil Antioxidantien in der Lage sind, die photochemische Reaktionskette zu unterbrechen, welche ausgelöst wird, wenn UV-Strahlung in die Haut eindringt. Neben dem erfindungsgemäßen Pflanzenextrakt sind weitere typische Beispiele hierfür Aminosäuren (z.B. Glycin, Alanin, Arginin, Serin, Threonin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin, Lutein) oder deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Disteärylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, Boldin, Boldo-Extrakt, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure. Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die UV-Lichtschutzfaktoren bzw. Antioxidantien können in Mengen von 0,01 bis 25, vorzugsweise 0,03 bis 10 und insbesondere 0,1 bis 5 Gew.-% bezogen auf die Gesamtmenge in den Zubereitungen, zugegeben werden.

Die erfindungsgemäßen Mittel und Extrakte aus *Pterocarpus marsupium* können weiterhin in schützenden und aufbauenden Pflegemitteln mit revitalisierenden und reaktivierenden Aktivitäten für die Haut verwendet werden. Diese Art der Verwendung dieser Pflegemittel, wirkt positiv beispielsweise gegen den negativen Einfluss der Umweltverschmutzung auf die Haut, indem sie die natürlichen Funktionen der Haut reaktivieren und die Haut widerstandsfähiger machen. Die Verwendung der erfindungsgemäßen Extrakte als schützende und aufbauende Pflegemittel ist prinzipiell für alle Zubereitungen möglich, die zur Prävention gegen Schädigungen oder bei Schädigungen der Haut und damit in der Hautpflege eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

Die erfindungsgemäßen Mittel können zur Herstellung von kosmetischen und/oder dermatologischen Zubereitungen, wie beispielsweise Schampoos, Haarspülungen, Haarkuren, Duschbäder, Cremes, Gele, Lotionen, alkoholische und wäßrig/alkoholische Lösungen, Emulsionen, Wachs/ Fett-Massen, Stiftpräparaten, Pudern oder Salben zum Einsatz kommen.

Diese Zubereitungen können ferner als weitere Hilfs- und Zusatzstoffe milde Tenside, Ölkörper, Emulgatoren, Periglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate: Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise da Dimethyldistearylammoniumchlorid, und Esterquats, insbesondere quatemierte Fettsäuretrialkanolaminestersalze: Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letzterevorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 **PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 **PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantiisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90). Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse); wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/ Acrylat-Copolymere, Octylacrylamid/Methylmeth-acrylat/tertButylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosm.Toil.108, 95 (1993**)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vor liegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**)**.

### Biogene Wirkstoffe

Unter biogenen Wirkstoffen sind im Rahmen der Erfindung zusätzlich solche zu verstehen, die nicht aus der Pflanze *Pterocarpus marsupium* stammen, wie beispielsweise Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Ceramide, Pseudoceramide, essentielle Öle, weitere Pflanzenextrakte und zusätzliche Vitaminkomplexe.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Filmbildner

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil.108, 95 (1993**)** entnommen werden.

### Insekten-Repellenben

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinaseinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglykol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methylolverbindungen, wie insbesondere Trimethylolethän, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetische Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

### Beispiele

### 1. Beispiel: Extraktion der Pflanzen mit destilliertem Wasser

20 destilliertes Wasser wurden auf 80 °C erhitzt und 4,5 kg zerkleinerte Pflanzen Pterocarpus marsupium wurden zugefügt. Die Mischung wurde 2 h stark gerührt und auf Raumtemperatur abgekühlt. Anschließend wurde 10 min bei einer Geschwindigkeit von 3500 g zentrifugiert. Die überstehende Flüssigkeit (Extrakt) war rot gefärbt. Zur Entfernung von unlöslichen Verunreinigungen wurde der Extrakt filtriert und über 72 h gefroren und zur Trockung entweder lyophilisiert oder sprühgetrocknet. Zur Spühtrockung wurde der Extrakt bei einer Anfangstemperatur von 185 °C und einer Endtemperatur von 80 °C sprühgetrocknet. Die Ausbeute an Extrakt betrug 3 bis 10 Gew.-% bezogen auf das Trockengewicht an eingesetzter Pflanze.

Eine weitere Möglichkeit der Aufarbeitung ist die Fraktionierung des Extraktes zum Beispiel über herkömmliche Chromatographiemethoden. Die Ausbeute an der Flavonderivate enthaltenden Fraktion lag bei 0,005 bis 0,5 Gew.-% - bezogen auf das Trockengewicht an eingesetzter Pflanze.

### 2. Beispiel: Extraktion der Pflanzen mit wässrigem Methanol

Beispiel 1 wurde wiederholt, die Extraktion jedoch mit 20 l 96 Gew.-%igen Ethanols durchgeführt. Die Extraktion wurde wie im Beispiel 1 durchgeführt und der Extrakt wie beschrieben weiter verarbeitet. Die Filtration wurde wie im Beispiel 1 beschrieben durchgeführt. Anschließend wurde zunächst der Alkohol bei 40 °C unter vermindertem Druck entfernt und durch destilliertes Wasser ausgetauscht. Zur Entfernung von unlöslichen Verunreinigungen wurde der Extrakt filtriert und über 72 h gefroren und zur Trockung entweder lyophilisiert oder sprühgetrocknet. Die Ausbeute an Extrakt betrug 2 bis 8 Gew.-% bezogen auf das Trockengewicht an eingesetzten Pflanzen. Die Ausbeute an Flavonderivate enthaltenden Fraktion lag bei 0,005 bis 0,5 Gew.-% - bezogen auf das Trockengewicht an eingesetzter Pflanze.

### 3. Toxizitätstest:

Das Ziel dieser Tests ist die Bestimmung der toxischen Konzentration des zu untersuchenden Mittels für Fibroblasten zur besseren Bestimmung der am besten wirksamen Konzentration. Der noch wirksame Konzentrationsbereich kann damit eingegrenzt werden.

Das zu untersuchende Mittel hat folgende Zusammensetzung (Gew.-%):
- Extrakt von *Pterocarpus marsupium* 5,00%
- Natrium Succinate 2,50%
- Glutaminsäure 0,50%
- Mannitol 92.00%

Es ist zum Beispiel unter dem Markennamen Trichodyn® erhältlich. Der Einfachheit halber wird in den folgenden Beispielen von Trichodyn® gesprochen, gemeint sind jedoch erfindungsgemäße Mittel, die eine oben erwähnte Zusammensetzung besitzen.

Methode: Effekte am Zellwachstum Humane Fibroblasten wurden in einem definiertem Nährmedium (DMEM = Dulbecco Minimum Essential Medium, Firma Life Technologie Sarl) mit 10 Gew.-% fötalem Kälberserum angeimpft und für 24 h bei 37 °C in einer 5 %igen CO₂-Atmosphäre inkubiert. Anschließend wurde das Nährmedium mit fötalem Kälberserum durch ein Nährmedium aus DMEM ohne fötalem Kälberserum ausgetauscht. Zu diesem Nährmedium wurden unterschiedliche

Konzentrationen an Aktivsubstanz in Form der oben angegebenen Zusammensetzung Trichodyn® gegeben. Nach einer drei tägigen Inkubation der Fibroblasten im Nährmedium wurde das Wachstum und die Stoffwechselakfivität beurteilt, indem der intrazelluläre Anteil an ATP nach der enzymatischen Lumineszenz-Methode von Vasseur (Journal français Hydrologie, 1981, 9, 149-156.) und der Anteil an Zellproteinen nach der Methode von Bradford (Anal. Biochem., 1976, 72,, 248-254) bestimmt wurde.

Die Bestimmung des Anteils an zellulären Proteinen erlaubt einen Rückschluß auf den Anteil an Makromolekülen wie Enzymen, Kollagen, Elastin oder anderen dermalen Makromolekülen, die zur Bildung von Bindegewebe notwendig sind. Der ATP-Anteil einer Zelle ist wichtig für viele Enzyme, deren Aktivität von diesem Energieüberträger abhängig ist.

Bestimmt wurde die letale Dosis des Trichodyn® bei der 50 % der untersuchten Fibroblasten nicht mehr lebensfähig waren (LD 50). Bis zu einer Konzentration von 0,1 Gew.-% zeigt Trichodyn® keine toxischen Effekte auf die humanen Fibroblasten. Der LD 50 - Wert lag bei der Bestimmung der Proteinanteile bei 0,53 Gew.-%. Bei der Bestimmung von ATP konnte dieser Wert für eine Konzentration von 0,67 Gew.-% von Trichodyn® ermittelt werden.

**Tabelle 1: Bestimmung der toxischen Konzentration von Trichodyn® durch Bestimmung des Proteinantells und des ATP-Anteils in humanen Fibroblasten**

| | **Proteine** | **ATP** |
|---|---|---|
| LD 50 (Gew.-%) | 0,53 | 0,67 |

### 4. Überlebensfähigkeit

Methode: Verbesserung der Überlebensfähigkeit Die Test wurden durchgeführt an humanen Fibroblasten. Der Test ermöglicht auf den ruhenden Zellen eine gewisse Anzahl von Parametern mengenmäßig zu bestimmen. Die Kultivierung der Zellen entspricht der Kultivierung aus der oben beschriebenen Methode, ausgenommen der Inkubationszeit. Die Inkubationszeit für diese Test betrugen 72 h. Die Überlebensfähigkeit wurde beurteilt über die Bestimmung der Schwefelreichen Proteine Metallothionein (MTT) und über die kolorimetrische Bestimmung des Anteils an Proteinen nach der Methode von Bradford (Anal. Biochem. 1976, 72, 248-254.) und über die Bestimmung des Anteils an Glutathion (GSH) mit einer fluoreszierenden Sonde, dem Orthophtalaldehyd nach der Methode von Hissin und Hilf (Anal. Biochem. 1976, 74, 214-216.). Das Glutathion (GSH) wird von Zellen produziert um direkt gegen oxidativen Stress und Umwelteinflüssen wie hoher Schwermetallbelastung reagieren zu können. Die drei Aminosäuren die im Glutathion gebunden vorliegen, werden durch spezifische Enzyme synthetisiert, welche ATP benötigen. Ein erhöhter Anteil an GSH steigert den Gehalt an Glutathion-S-transferase, einem Enzym, welches zur Entgiftung benötigt wird. Ein erhöhter Anteil an reduziertem Glutathion nach Behandlung der Zellen mit Trichodyn®, ist demnach ein Maß für die gesteigerte Überlebensfähigkeit der Zelle bei äußeren Stress und Belastungsbedingungen. Die Tests wurden dreifach durchgeführt und dann zweimal wiederholt. Die Ergebnisse wurden ermittelt in Prozent im Vergleich zur Kontrolle.

**Tabelle 2: Bestimmung des Proteinanteils und des GSH-Anteils in humanen Fibroblasten**

| **Konzentration in Gew.-%** | **MTT** | **Anteil an Proteine in %** | **Anteil an GSH in %** |
|---|---|---|---|
| 0 | 100 | 100 | 100 |
| 0,6 | 90 | 92 | 134 |

Die Ergebnisse zeigen, dass Trichodyn®, bei einer Konzentration von 0,6 Gew.-% den Anteil an zellulärem Glutathion erhöht hat und somit unter anderem gegen oxidativen Stress für Haut oder Haare eingesetzt werden kann.

### 5. Zellschutzwirkung gegen UVB an in vitro gezüchteten menschlichen Keratinozyten

Hintergrund: UVB-Strahlen lösen durch Aktivierung eines Enzyms, nämlich Phospholipase A2 oder PLA2, welche Arachidonsäure aus den Phospholipiden der Plasmamembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet. Eine durch UV-Strahlung belastete Zelle produziert einen weiteren Entzündungs-Mediator, das Interleukin-1-alpha (1'ILα), welches in der Lage ist, die Ausscheidung von Prostaglandinen zu induzieren.

Methode: Der Effekt von UVB-Strahlung wurde an Keratinocyten in vitro untersucht indem die Freisetzung des Cytoplasaenzyms LDH (Lactat Dehydrogenase) und der Gehalt an (1'ILα) bestimmt wurde.

Zur Durchführung der Tests wurde ein definiertes Medium, das fötales Kälberserum enthält, mit den Keratinozyten beimpft und Trichodyn® in bestimmten Konzentrationen 72 Stunden nach dem Beimpfen zugegeben.

Die Keratinozyten wurden sodann mit einer UVB-Dosis bestrahlt (50 mJ/cm² - Röhren: DUKE GL40E).

Nach weiterer 1 tägiger Inkubation bei 37 °C und bei 5 % CO₂ wurde der LDH-Gehalt im Überstand bestimmt. Der Gehalt von LDH- (Lactatdehydrogenase) wurde mittels einer Enzymreaktion bestimmt (verwendetes kit zur Untersuchung des LDH Gehaltes von der Firma Roche). Desweiteren wurde der Gehalt an (1'ILα) durch den ELISA Test bestimmt. Die Anzahl adhärenter Keratinozyten wird (nach Trypsinbehandlung) mit einem Partikelzählgerät bestimmt.

**Tabelle 3: Zellschutzwirkung gegen UVB-Strahlen; Ergebnisse in % bezogen auf die Kontrolle, Mittelwert aus 2 Versuchen, jeder mit zwei Wiederholungen**

| | **Anzahl Keratinocyten** | **Gehalt an freigesetzter LDH** | **Gehalt an (1'ILα)** |
|---|---|---|---|
| Kontrolle ohne UV | 100 | 0 | 0 |
| UVB | 17 | 100 | 100 |
| UVB + Trichodyn® 0,1 % | 26 | 58 | 70 |
| UVA + Trichodyn® 0,2 % | 26 | 43 | 72 |
| UVB + Aspirin | 15 | 66 | 69 |

Die Ergebnisse dieser Tests belegen, dass ein erfindungsgemäßes Mittel den Effekt von UVB-Strahlung auf die Anzahl an Keratinocyten, auf den Gehalt an (1'ILα) und an freigesetzte LDH reduziert. Die beschriebenen Mittel zeigen demnach die Fähigkeit, die durch UVB-Strahlung hervorgerufene Schädigung an Zellmembranen zu reduzieren.

### 6. Beispiel: Anti-Glykosylierungstest an Typ I Kollagen

Hintergrund: Die nicht-enzymatische Glykosylierung der Proteine ist ein entscheidender Vorgang im Altem des menschlichen Gewebes und erklärt die Retikulation der ausser-zellulären Matrix und der Basalmembran. Durch die Glykosylierung an dermalen Proteinen mit reduzierenden Zuckern wie Glukose, Fructose oder Saccharose entstehen langlebige Schiffse Basen. Dieser Mechanismus zerstört die extra-zelluläre Matrix-Struktur und den Metabolismus der Fibroblasten. Außerdem katalysieren die Schiffsen Basen die Herstellung reaktiver Formen des Sauerstoffs, die die Wirkungen der nicht-enzymatischen Glykosylierung besonders durch UVA-Strahlung noch verstärken können. Desweiteren fördert der oxidativen Stress eine Stabilisierung der Schiffs Basen bis hin zu den sogenannten AGE's (Advanced Glycated Endproducts) welche sich in der Haut anreichern.

Methode: Die in-tubo Test wurden auf Kollagen des Typs I vorgenommen, welches 21 Tage bei 45 °C in Gegenwart von 1 %iger Glykose inkubiert wurde. Der Gehalt an Schiffs Basen wrude durch Fluoreszenz bei 430 nm (Exitation bei 350 nm) am ersten und am letzten Tag der 21-tägigen Inkubation bestimmt.

**Tabelle 4: Fluoreszenz Bestimmung von Kollagen nach 21 tägiger Inkubation mit Glucose**

| | **Fluoreszenz / Kontrolle am 21. Tag** |
|---|---|
| Kontrolle ohne Glucose | 47 |
| Kontrolle mit Glucose | 100 |
| Glucose + Trichodyn® (0,3 Gew.-%) | 81 |
| Glucose + Trichodyn® (1 Gew.-%) | 36 |
| Glucose + Trichodyn® (3 Gew.-%) | 24 |

Die Ergebnisse zeigen eindrucksvoll, dass Trichodyn® deutlich die Glykosylierung von Kollagen Typ I reduziert. Bereits bei einer Konzentration von 0,3 Gew.-% liegt im Vergeich zur Kontrolle mit Glucose eine verringerte Glykosylierung vor. Der ermittelte IC50 Wert (Konzentration an Trichodyn®, bei der 50 % Glykosylierung inhibiert wurden) lag bei 0,4 Gew.-%.

### 7. Klinische Studie

Die klinische Studie wurde mit 30 männlichen Probanden mit diffuser androgener Alopezie (Typ I bis III) durchgeführt. 15 Probanden wurden mit Placebo-Lotion behandelt, 15 Probanden erhielten Lotion mit 10% Wirkstoff (Trichodyn®). Der Auswahltest bestand aus einem Trichogramm, in dem die Rate A/T (Haar in der anagenen Phase/ Haar in der telogenen Phase) an mindestens einer Stelle der untersuchten Kopfhaut unter 3 sein mußte.

### Methode:

Über einen Zeitraum von 6 Monaten wurden dreimal wöchentlich die gesamte Kopfhaut und Haare mit 10 ml der Lotio eingerieben.

Die Anti-Haarausfall-Aktivität wurde quantitativ durch ein Trichogramm nach Bouhanna P., Reygagne P.: Pathologie du cheveu. Masson, Paris, 1999 ermittelt. Das Trichogramm wurde an zwei Stellen der Kopfhaut (frontal und occipital) jeweils vor der Behandlung und nach 2, 4 und 6 Monaten aufgenommen, wobei eine standardisierte Probennahme von 30 bis 50 Haaren zur Bestimmung des Anteils an telogenen Haaren (T) erfolgte. Bestimmt wurde die Entwicklung telogener Haare in % bezogen auf den Basiswert (=100), der sich aus dem Mittelwert der Bestimmung in der jeweiligen Probandengruppe und auf der jeweiligen Kopfhautfläche (frontal, occipital) am Tag 0 ergibt.

Dabei ist zu berücksichtigen, dass Haarausfall auch jahreszeitabhängig ist, im Februar und März fällt er geringer aus und nimmt dann von März bis Ende Juli zu (Randall VA, Ebling FJG: Seasonal changes in human hair growth. British Journal of Dermatology, 124:146-151,1991). Der klinische Test begann in der Phase geringen Haarausfalls und endete in der hochaktiven telogenen Phase.

Die Abnahme des prozentualen Anteils telogener Haare korrespondiert mit der Abnahme der Haarausfall-Aktivität.

Getestetes Produkt (Mengenangaben in Gew. % - bezogen auf die Formierung):

| | |
|---|---|
| Wasser | 78,78 |
| Wirkstoff (Trichodyn®, siehe 3. Toxizitätstest) | 10,00 |
| Alkohol | 7,80 |
| Elestab® 50 J | 0,27 |
| PEG-40 Hydrogenated castor oil | 0,30 |
| Duftstoffe | 0,10 |
| Acrylates / Steareth-20 Itaconate Copolymer | 1,25 |
| NaOH (N) | 1,50 |

In der Placebo-lotion wurde der Anteil an Wirkstoff durch Wasser ersetzt.

### Ergebnisse:

**Tabelle 5a: % Anteil telogener Haare der Frontalseite nach 2, 4 und 6-monatiger Behandlung bezogen auf den Wert zum Studienbeginn Tag 0 (=100), Mittelwert: n=15 Probanden**

| | **Tag 0** | **Tag 56** | **Tag 112** | **Tag 168** |
|---|---|---|---|---|
| Placebo Lotion | 100 | 120 | 118 | 110 |
| Lotion mit 10 Gew. % Trichodyn® | 100 | 99 | 85 | 101 |

**Tabelle 5b: % Anteil telogener Haare der occipitalen Seite nach 2, 4 und 6-monatiger Behandlung bezogen auf den Wert zum Studienbeginn Tag 0 (=100), Mittelwert: n=15 Probanden**

| | **Tag 0** | **Tag 56** | **Tag 112** | **Tag 168** |
|---|---|---|---|---|
| Placebo Lotion | 100 | 98 | 142 | 169 |
| Lotion mit 10 Gew. % Trichodyn® | 100 | 112 | 94 | 118 |

Nach monatiger Behandlung mit Placebo-Lotion hat der %-Anteil der Haare in der telgogenen Phase an beiden untersuchten Kopfhautstellen, insbesondere jedoch in der Occipital-Gegend, signifikant zugenommen, was der üblichen Zunahme der Alopezie im Sommer entspricht. Nach Behandlung mit Trichodyn® zeigt sich jedoch eine deutliche Reduktion des Haarausfalls, die zu einem Erhalt des Haupthaares führt.

### 8. Beispielrezepturen kosmetischer Mittel mit Trichodyne®

Die erfindungsgemäßen Mittel wurden in den folgenden erfindungsgemäßen Rezepturen K1 bis K21 sowie 1 bis 30 eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die erfindungsgemäßen Mittel stabil gegen oxidative Zersetzung.

**Tabelle 6: Softcreme Rezepturen K1 bis K7**

| (Alle Angaben in Gew.-% bezogen auf das kosmetische Mittel) | | | | | | | | |
|---|---|---|---|---|---|---|---|---|
| INCI Bezeichnung | K1 | K2 | K3 | K4 | K5 | K6 | K7 | V1 |
| Glyceryl Stearate (and) Ceteareth-12/20 | 8,0 | 8,0 | 8,0 | 8,0 | 8,0 | 8,0 | 8,0 | 8,0 |
| (and) Cetearyl Alcohol (and) Cetyl | | | | | | | | |
| Palmitate | | | | | | | | |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyl Isononanoate | 3,0 | 3,0 | 3,0 | 3,0 | 3,0 | 3,0 | 3,0 | 3,0 |
| Glycerin (86 Gew.%ig) | 3,0 | 3,0 | 3,0 | 3,0 | 3,0 | 3,0 | 3,0 | 3,0 |
| Trichodyn® | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 7: Nachtcremerezepturen K8 bis K14**

| (Alle Angaben in Gew.-% bezogen auf das kosmetische Mittel) | | | | | | | | |
|---|---|---|---|---|---|---|---|---|
| INCI Bezeichnung | K8 | K9 | K10 | K11 | K12 | K13 | K14 | V2 |
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryl Isononanoate | 8,0 | 8,0 | 8,0 | 8,0 | 8.0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| Trichodyn® | 0,5 | 0,5 | 0,5 | 0,5 | 0,5 | 0,5 | 0,5 | |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 8: W/O Bodylotion Rezepturen K15 bis K21**

| (Alle Angaben in Gew.-% bezogen auf das kosmetische Mittel) | | | | | | | | |
|---|---|---|---|---|---|---|---|---|
| INCI-Bezeichnung | K15 | K16 | K17 | K18 | K19 | K20 | K21 | V3 |
| PEG-7 Hydrogenated Castor Oil | 7,0 | 7,0 | 7,0 | 7,0 | 7,0 | 7,0 | 7,0 | 7,0 |
| Decyl Oleate | 7,0 | 7,0 | 7,0 | 7,0 | 7,0 | 7,0 | 7,0 | 7,0 |
| Cetearyl Isononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5.0 | 5,0 | 5,0 |
| MgSO₄ · 7 H₂O | 1,0 | 1,0 | 1,0 | 1,0 | 1,0 | 1,0 | 1,0 | 1,0 |
| Trichodyn® | 1,5 | 1,5 | 1,5 | 1,5 | 1,5 | 1,5 | 1,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | | | |
| Panthenol | | | | | | | 0,5 | |
| Wasser | Ad 100 | | | | | | | |

| | | | | | | | | |
|---|---|---|---|---|---|---|---|---|
| ¹⁾ Desoxyribonucleinsäure: Molekuargewicht ca. 70000, Reinheit (bestimmt durch spektro-photometrische Messung der Absorption bei 260 nm sowie 280 nm): mindestens 1,7. | | | | | | | | |

**Tabelle 9**

| **Kosmetische Zubereitungen** (Alle Angaben in Gew.-% bezogen auf das kosmetische Mittel, Wasser, Konservierungsmittel addieren sich zu 100 Gew.-%) | | | | |
|---|---|---|---|---|
| **Zusammensetzung (INCI)** | **1 Gew.-%** | **2 Gew.-%** | **3Gew.-%** | **4 Gew.-%** |
| **Texapon® NSO** | 38,0 | 38,0 | 25,0 | - |
| Sodium Laureth Sulfate | | | | |
| **Texapon®SB3** | - | - | 10,0 | - |
| Disodium Laureth Sulfosuccinate | | | | |
| **Plantacare®818** | 7,0 | 7,0 | 6,0 | - |
| Coco Glucosides | | | | |
| **Plantacare® PS 10** | - | - | - | 20,0 |
| Sodium Laureth Sulfate (and) Coco Glucosides | | | | |
| **Dehyton® PK 45** | - | - | 10,0 | |
| Cocamidopropyl Betaine | | | | |
| **Lamesoft® PO 65** | 3,0 | | | 4,0 |
| Coco-Glucosid (and) Glyceryl Oleate | | | | |
| **Lamesoft® LMG** | - | 5,0 | - | |
| Glyceryl Laurate (and) Potassium Cocoyl Hydrolyzed Collagen | | | | |
| **Euperlan® PK 3000 AM** | - | 3,0 | 5,0 | 5,0 |
| Glycol Distearate (and) laureth-4 (and) Cocamidopropyl Betaine | | | | |
| **Trichodyn®** | 1,0 | 1,0 | 1,0 | 1,0 |
| **Arlypon®** F | 3,0 | 3,0 | 1,0 | - |
| Laureth-2 | | | | |
| **Sodium Chloride** | - | 1,5 | - | 1,5 |

| | | | | |
|---|---|---|---|---|
| (1-2) Duschbad. (3) Duschgel, (4) Waschlotion | | | | |

**Tabelle 10**

| **Kosmetische Zubereitungen Duschbad "Two in One"** (Alle Angaben in Gew.-% bezogen auf das kosmetische Mittel, Wasser, Konservierungsmittel addieren sich zu 100 Gew.-%) | | | | |
|---|---|---|---|---|
| **Zusammensetzung (INCI)** | **5** | **6** | **7** | **8** |
| **xapon® NSO** | 30,0 | 25,0 | | 25,0 |
| Sodium Laureth Sulfate | | | | |
| **Plantacare® 818** | | | | 8,0 |
| Coco Glucosides | | | | |
| **Plantacare® 2000** | | 8,0 | | |
| Decyl Glucoside | | | | |
| **Plantacare® PS 10** | | | 20,0 | |
| Sodium Laureth Sulfate (and) Coco Glucosides | | | | |
| **Dehyton® PK 45** | | 10,0 | 10,0 | |
| Cocamidopropyl Betaine | | | | |
| **Lamesoft® PO 65** | 5,0 | | | |
| Coco-Glucosid (and) Glyceryl Oleate | | | | |
| **Lamesoft® LMG** | | 5,0 | 5,0 | |
| Glyceryl Laurate (and) Potassium Cocoyl Hydrolyzed Collagen | | | | |
| **Gluadin® WQ** | 3,0 | | | |
| Laurdimonium Hydroxapropyl] Hydrolyzed Wheat Protein | | | | |
| **Gluadin® WK** | | | | |
| Sodium Cocoyl Hydrolyzed Wheat Protein | | | | |
| **Euperlan® PK 3000 AM** | 5,0 | 3,0 | 4,0 | - |
| Glycol Distearate (and) Laureth-4 (and) Cocamidopropyl Betaine | | | | |
| **Panthenol** | 0,5 | - | - | 0,5 |
| **Trichodyn®** | 1,0 | 1,0 | 1,0 | 1,0 |
| **Arlypon®** F | 2,6 | 1,6 | | 1,0 |
| Laureth-2 | | | | |
| **Sodium Chloride** | - | - | - | - |

**Tabelle 11: Kosmetische Zubereitungen (Alle Angaben in Gew-% bezogen auf das kosmetische Mittel, Wasser, Konservierungsmittel addieren sich zu 100 Gew-%)**

| **Zusammensetzung (INCI)** | **9** | **10** | **11** | **12** | **3** | **14** | **15** | **16** | **17** | **18** |
|---|---|---|---|---|---|---|---|---|---|---|
| **Dehymuls® PGPH** | 4,0 | 3,0 | - | 5,0 | - | - | - | - | - | - |
| Polyglyceryl-2 Dipolyhydroxystearate | | | | | | | | | | |
| **Lameform® TGI** | 2,0 | 1,0 | - | - | - | - | - | - | - | - |
| Polyglyceryl-3 Diisostearate | | | | | | | | | | |
| **Emulgade® PL 68150** | - | - | - | - | 4,0 | - | - | - | 3,0 | - |
| Cetearyl Glucoside (and) Cetearyl Alcohol | | | | | | | | | | |
| **Eumuligin®B2** | - | - | - | - | - | - | - | 2,0 | - | - |
| Ceteareth-20 | | | | | | | | | | |
| **Tegocare® PS** | - | - | 3,0 | - | - | - | 4,0 | - | - | - |
| Polyglyceryl-3 Methylglucose Distearate | | | | | | | | | | |
| **Eumulgin VL 75** | - | - | - | - | - | 3,5 | - | - | 2,5 | - |
| Polyglyceryl-2 Dipolyhydroxystearate (and) Lauryl Glucoside (and) | | | | | | | | | | |
| Glycerin | | | | | | | | | | |
| **Bees Wax** | 3,0 | 2,0 | 5,0 | 2,0 | - | - | - | - | - | - |
| **Cutina® GMS** | - | - | - | - | - | 2,0 | 4,0 | - | - | 4,0 |
| Glyceryl Stearate | | | | | | | | | | |
| **Lanette® 0** | - | - | 2,0 | - | 2,0 | 4,0 | 2,0 | 4,0 | 4,0 | 1,0 |
| Cetearyl Alcohol | | | | | | | | | | |
| **Antaron® V 216** | - | - | - | - | - | 3,0 | - | - | - | 2,0 |
| PVP / Hexadecene Copolymer | | | | | | | | | | |
| **Myritol® 818** | 5,0 | - | 10,0 | - | 8,0 | 6,0 | 6,0 | - | 5,0 | 5,0 |
| Cocoglycerides | | | | | | | | | | |
| **Finsolv® TN** | - | 6,0 | - | 2,0 | - | - | 3,0 | - | - | 2,0 |
| C12/15 Alkyl Benzoate | | | | | | | | | | |
| **Cetiol® J 600** | 7,0 | 4,0 | 3,0 | 5,0 | 4,0 | 3,0 | 3,0 | - | 5,0 | 4,0 |
| Oleyl Erucate | | | | | | | | | | |
| **Cetiol® OE** | 3,0 | - | 6,0 | 8,0 | 6,0 | 5,0 | 4,0 | .3,0 | 4,0 | 6,0 |
| Dicaprylyl Ether | | | | | | | | | | |
| **Mineral Oil** | - | 4,0 | - | 4,0 | - | 2,0 | - | 1,0 | - | - |
| **Cetiol® PGL** | - | 7,0 | 3,0 | 7,0 | 4,0 | - | - | - | 1,0 | - |
| Hexadecanol (and) Hexyldecyl Laurate | | | | | | | | | | |
| **Panthenol Bisabolol** | 1,2 | 1,2 | 1,2 | 1,2 | 1,2 | 1,2 | 1,2 | 1,2 | 1,2 | 1,2 |
| Trichodyn® | 1,0 | 1,0 | 1,0 | 1,0 | 1,0 | 1,0 | 1,0 | 1,0 | 1,0 | 1,0 |
| **Copherol® F 1300** | 0,5 | 1,0 | 1,0 | 2,0 | 1,0 | 1,0 | 1,0 | 2,0 | 0,5 | 2,0 |
| Tocopherol / Tocopheyl Acetate | | | | | | | | | | |
| **Neo Heliopan® Hydro** | 3,0 | - | - | 3,0 | - | - | 2,0 | - | 2,0 | - |
| Sodium Phenylbenzimidazole Sulfonate | | | | | | | | | | |
| **Neo Heliopan® 303** | - | 5,0 | - | - | - | 4,0 | 5,0 | - | - | 10,0 |
| Octocrylene | | | | | | | | | | |
| **Neo Heliopan® BB** | 1,5 | - | - | 2,0 | 1,5 | - | - | - | 2,0 | - |
| Benzophenone-3 | | | | | | | | | | |
| **Neo Heliopan® E 1000** | 5,0 | - | 4,0 | - | 2,0 | 2,0 | 4,0 | 10,0 | - | - |
| **Isoamyl p-Methoxycinnamate** | | | | | | | | | | |
| **Neo Heliopan® AV** | 4,0 | - | 4,0 | 3,0 | 2,0 | 3,0 | 4,0 | - | 10,0 | 2,0 |
| Octyl Methoxycinnamate | | | | | | | | | | |
| **Uvinul® T 150** | 2,0 | 4,0 | 3,0 | 1,0 | 1,0 | 1,0 | 4,0 | 3,0 | 3,0 | 3,0 |
| Octyl Triazone | | | | | | | | | | |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |

| | | | | | | | | | | |
|---|---|---|---|---|---|---|---|---|---|---|
| (14) W/O-Sonnenschutzcreme, (15-17) W/O-Sonnenschutzlotion, (18, 21, 23) O/W-Sonnenschutzlotion (19, 20, 22) O/W-Sonnenschutzcreme | | | | | | | | | | |

**Tabelle 12: Kosmetische Zubereitungené (Alle Angaben in Gew.-% bezogen auf das kosmetische Mittel, Wasser, Konservierungsmittel addieren sich zu 100 Gew.-%)**

| **Zusammensetzung (INCI)** | **19 Gew.-%** | **20 Gew.-%** | **21 Gew.-%** | **22 Gew.-%** | **23 Gew.-%** | **24 Gew.-%** |
|---|---|---|---|---|---|---|
| **Dehyquart® A** | 4,0 | 4,0 | | | 3,0 | |
| Cetrimonium Chloride | | | | | | |
| **Dehyquart L® 80** | | | 1,2 | 1,2 | | 1,0 |
| Dicocoylmethylethoxymonium Methosulfate (and) Propylenglycol | | | | | | |
| **Eumulgin® B2** | 0.8 | | - | 0,8 | - | 1,0 |
| Ceteareth-20 | | | | | | |
| **Eumulgin® VL 75** | - | 2,0 | 2,0 | - | 0.8 | - |
| Lauryl Glucoside (and) Polyglyceryl-2 Polyhydroxystearate (and) Glycerin | | | | | | |
| **Lanette® 0** | 3,0 | 3,0 | 3,0 | 3,0 | 3,0 | 3,0 |
| Cetearyl Alcohol | | | | | | |
| **Cutina® GMS** | - | 0,5 | - | 0,5 | - | 1,0 |
| Glyceryl Stearate | | | | | | |
| **Lamesoft® PO 65** | | - | 3,0 | - | - | 3,0 |
| Coco-Glucosid (and) Glyceryl Oleate | | | | | | |
| **Cetiol® J 600** | - | 0,5 | - | 1,0 | - | 1,0 |
| Oleyl Erucate | | | | | | |
| **Eutanol® G** | - | - | 1,0 | - | - | 1,0 |
| Octyldodecanol | | | | | | |
| **Nutrilan® Keratin W** | 5,0 | - | - | 2,0 | - | - |
| Hydrolyzed Keratin | | | | | | |
| **Generol® 122 N** | - | - | - | - | 1,0 | 1,0 |
| Soja Sterol | | | | | | |
| **Trichodyn®** | 1,0 | 1,0 | 1,0 | 1,0 | 1,0 | 1,0 |
| **Copherol® 1250** | - | - | 0,1 | 0,1 | - | - |
| Tocopheryl Acetate | | | | | | |

| | | | | | | |
|---|---|---|---|---|---|---|
| (24-27) Haarspülung, (28-29) Haarkur | | | | | | |

**Tabelle 13: Kosmetische Zubereitungen Shampoo (Alle Angaben in Gew.-% bezogen auf das kosmetische Mittel, Wasser, Konservierungsmittel addieren sich zu 100 Gew.-%)**

| Z**usammensetzung (INCI)** | **25** | **26** | **27** | **28** | **29** | **30** |
|---|---|---|---|---|---|---|
| **Texapon® NSO** | 30,0 | | | 30,0 | 25,0 | |
| Sodium Laureth Sulfate | | | | | | |
| **Texapon® K 14 S** | | 30,0 | | | | 30,0 |
| Sodium Myreth Sulfate | | | | | | |
| **Texapon® SB** 3 | | 10,0 | | | | |
| Disodium Laureth Sulfosuccinate | | | | | | |
| **Plantacare® 818** | 4,0 | | | | | |
| Coco Glucosides | | | | | | |
| **Plantacare® 2000** | | 4,0 | | | | |
| Decyl Glucoside | | | | | | |
| **Plantacare® PS 10** | | | 20,0 | | | |
| Sodium Laureth Sulfate (and) Coco Glucosides | | | | | | |
| **Dehyton® PK 45** | 5,0 | | | 10,0 | | 10,0 |
| Cocamidopropyl Betaine | | | | | | |
| **Gluadin® WK** | | | | | 8,0 | |
| Sodium Cocoyl Hydrolyzed Wheat Protein | | | | | | |
| **Lamesoft® PO 65** | - | - | - | - | 2,0 | 2,0 |
| Coco-Glucosid (and) Glyceryl Oleate | | | | | | |
| **Nutrilan® Keratin W** | 5,0 | - | - | - | | - |
| Hydrolyzed Keratin | | | | | | |
| **Gluadin® W 40** | - | 2,0 | - | 2,0 | - | - |
| Hydrolyzed Wheat Protein | | | | | | |
| **Euperlan® PK 3000 AM** | - | - | - | 3,0 | 3,0 | - |
| Glycol Distearate (and) Laureth-4 (and) Cocamidopropyl Betaine | | | | | | |
| **Panthenol** | - | - | - | - | - | 0,2 |
| **Trichodyn®** | 1,0 | 1,0 | 1,0 | 1,0 | 1,0 | 1,0 |
| **Arlypon® F** | 1,5 | - | - | - | - | - |
| Laureth-2 | | | | | | |
| **Sodium Chloride** | - | 1,6 | 2,0 | 2,2 | - | 3,0 |

| | | | | | | |
|---|---|---|---|---|---|---|
| Alle in den Tabellen 6-13 aufgeführten und verwendeten Substanzen mit registriertem Warenzeichen ® sind Marken und Produkte der COGNIS Gruppe. | | | | | | |

## Patentansprüche

1. Kosmetische und/oder pharmazeutische Mittel, enthaltend
(a) eine wirksame Menge eines Extraktes aus Pterocarpus marsupium und
(b) Dicarbonsäuren und/oder deren Salze und/oder Aminosäuren

2. Mittel nach Anspruch 1, **dadurch gekennzeichnet, dass** die Extrakte ganz oder überwiegend als Wirkstoffe Flavonderivate enthalten.

3. Mittel nach Anspruch 1, **dadurch gekennzeichnet, dass** die Mittel Dicarbonsäuren enthalten, die ausgewählt sind aus der Gruppe, die gebildet wird von Oxalsäure, Malonsäure, Bernsteinsäure und Glutarsäure.

4. Mittel nach einem der Ansprüche 1 oder 2, **dadurch gekennzeichnet, daß** die Mittel Salze von Dicarbonsäuren gemäß Anspruch 3 enthalten, die ausgewählt sind aus der Gruppe die gebildet wird aus Alkali- und Erdalkalisalzen, insbesondere aus Natrium-, Kalium, Magnesium- und Calciumsalzen.

5. Mittel nach mindestens einem der Ansprüche 1 bis 4, **dadurch gekennzeichnet, daß** die Mittel Aminosäuren enthalten, die ausgewählt sind aus der Gruppe, die gebildet wird von Glycin, Alanin, Leucin, Isoleucin, Serin, Threonin, Cystein, Asparaginsäure, Glutaminsäure, Asparagin, Glutamin, Phenylalanin, Tyrosin, Methionin, Valin, Prolin, Lysin und Histidin.

6. Mittel nach mindestens einem der Ansprüche 1 bis 6, **dadurch gekennzeichnet, daß** sie
(a) 0,001 bis 25 Gew.-% Extrakt und
(b) 0,001 bis 15 Ges.-% Dicarbonsäuren und/oder deren Salze und/oder 0,0005 bis 10 Gew.-% Aminosäuren
enthalten, mit der Maßgabe, dass sich gegebenenfalls die Mengenangaben mit Wasser und/oder weiteren Hilfs- und Zusatzstoffen zu 100 Gew.-% ergänzen.

7. Kosmetische Verwendung von Mitteln nach einem der Ansprüche 1 bis 6
gegen Haarausfall
oder gegen die Hautalterung
oder gegen oxidativen Stress für Haar.

8. Kosmetische Verwendung von Pterocarpus marsupium Extrakten
gegen Haarausfall,
oder gegen die Hautalterung
oder gegen oxidativen Stress für Haar.

9. Mittel nach einem der Ansprüche 1 bis 6 gegen androgene Alopezia, oder als UV/IR-Lichtschutzmittel, oder als Mittel gegen die zchädigung von Fibroblasten und/oder Keratinocyten durch UV-Strahlung, oder gegen oxidativen Stress für Haut.

10. Pterocarpus marsupium Extrakte gegen androgene Alopezia, oder als UV/IR Lichtschutzmittel, oder als Mittel gegen die Schädigung von Fibroblasten und/oder Keratinocyten durch UV-Strahlung, odes gegen oxidativen Stress für Haut.

## Claims

1. Cosmetic and/or pharmaceutical preparations containing
(a) an effective quantity of an extract of Pterocarpus marsupium and
(b) dicarboxylic acids and/or salts thereof and/or amino acids

2. Preparations as claimed in Claim 1, **characterized in that** the extracts exclusively or predominantly contain flavone derivatives as active ingredients.

3. Preparations as claimed in Claim 1, **characterized in that** they contain dicarboxylic acids selected from the group consisting of oxalic acid, malonic acid, succinic acid and glutaric acid.

4. Preparations as claimed in Claim 1 or 2, **characterized in that** they contain salts of the dicarboxylic acids mentioned in Claim 3 selected from the group consisting of alkali metal and alkaline earth metal salts, more particularly sodium, potassium, magnesium and calcium salts.

5. Preparations as claimed in at least one of Claims 1 to 4, **characterized in that** they contain amino acids selected from the group consisting of glycine, alanine, leucine, isoleucine, serine, threonine, cysteine, aspartic acid, glutamic acid, asparagine, glutamine, phenylalanine, tyrosine, methionine, valine, proline, lysine and histidine.

6. Preparations as claimed in at least one of Claims 1 to 5, **characterized in that** they contain
(a) 0.001 to 25% by weight extract and
(b) 0.001 to 15% by weight dicarboxylic acids and/or salts thereof and/or 0.0005 to 10% by weight amino acids,
with the proviso that the quantities shown optionally add up to 100% by weight with water and/or other auxiliaries and additives.

7. Cosmetic use of the preparations claimed in one of Claims 1 to 6 against hair loss or ageing of skin or oxidative hair stress.

8. Cosmetic use of Pterocarpus marsupium extracts against hair loss or ageing of skin or oxidative hair stress.

9. Preparations as claimed in Claims 1 to 6 against androgenic alopecia, or as UV/IR protection preparations, or as preparations against the damaging of fibroblasts and/or keratinocytes by UV radiation, or against oxidative skin stress.

10. Pterocarpus marsupium extracts against androgenic alopecia, or as UV/IR protection preparations, or as preparations against the damaging of fibroblasts and/or keratinocytes by UV radiation, or against oxidative skin stress.

## Revendications

1. Produits cosmétiques et/ou pharmaceutiques, contenant
a) une quantité efficace d'un extrait de *Pterocarpus marsupium* et
b) des acides dicarboxyliques et/ou leurs sels et/ou des acides aminés.

2. Produits selon la revendication 1, **caractérisés en ce que** les extraits contiennent en totalité ou en majorité en tant que substances actives des dérivés de flavones.

3. Produits selon la revendication 1, **caractérisés en ce que** les produits contiennent des acides dicarboxyliques qui sont choisis dans le groupe qui est constitué par l'acide oxalique, l'acide malonique, l'acide succinique et l'acide glutarique.

4. Produits selon la revendication 1 ou 2, **caractérisés en ce que** les produits contiennent des sels d'acides dicarboxyliques selon la revendication 3, qui sont choisis dans le groupe qui est constitué par des sels alcalins et alcalino-terreux, en particulier les sels de sodium, potassium, magnésium et calcium.

5. Produits selon au moins l'une des revendications 1 à 4, **caractérisés en ce que** les produits contiennent des acides aminés qui sont choisis dans le groupe qui est constitué par la glycine, l'alanine, la leucine, l'isoleucine, la sérine, la thréonine, la cystéine, l'acide aspartique, l'acide glutamique, l'asparagine, la glutamine, la phénylalanine, la tyrosine, la méthionine, la valine, la proline, la lysine et l'histidine.

6. Produits selon au moins l'une des revendications 1 à 5, **caractérisés en ce qu'**ils contiennent
(a) de 0,001 à 25 % en poids d'extrait et
(b) de 0,001 à 15 % en poids d'acides dicarboxyliques et/ou de leurs sels et/ou 0,0005 à 10 % en poids d'acides aminés,
étant entendu que les données quantitatives éventuellement avec de l'eau et/ou d'autres adjuvants et additifs se complètent à 100 % en poids.

7. Utilisation cosmétique de produits selon l'une quelconque des revendications 1 à 6
contre la chute des cheveux
ou contre le vieillissement de la peau
ou contre le stress oxydant pour le cheveu.

8. Utilisation cosmétique d'extraits de *Pterocarpus marsupium*
contre la chute des cheveux
ou contre le vieillissement de la peau
ou contre le stress oxydant pour le cheveu.

9. Produits selon l'une quelconque des revendications 1 à 6, contre l'alopécie androgénétique, ou en tant que photoprotecteur UV/IR, ou en tant qu'agents contre l'endommagement de fibroblastes et/ou de kératinocytes par le rayonnement UV, ou contre le stress oxydant pour la peau.

10. Extraits de *Pterocarpus marsupium* contre l'alopécie androgénétique, ou en tant que photoprotecteur UV/IR, ou en tant qu'agents contre l'endommagement de fibroblastes et/ou de kératinocytes par le rayonnement UV, ou contre le stress oxydant pour la peau.
